# EUROPEAN PATENT SPECIFICATION

(11) **EP 1 397 681 B1**
(45) Date of publication and mention of the grant of the patent: **14.03.2007**
(21) Application number: 02739056.6
(22) Date of filing: 18.06.2002
(51) Int. Cl.: G01N 33/573

(54) **METHOD FOR IDENTIFICATION OF AGENTS FOR THE TREATMENT OF DIABETES**
VERFAHREN ZUR IDENTIFIZIERUNG VON MITTELN ZUR BEHANDLUNG VON DIABETES
METHODES D'IDENTIFICATION D'AGENTS POUR LE TRAITEMENT DES DIABETES

(30) Priority: 18.06.2001 SE 0102147
(43) Date of publication of application: 17.03.2004
(73) Proprietor: BIOVITRUM AB, 112 76 Stockholm (SE)
(72) Inventor: BENDZ, Christina, S-178 38 EKERÖ (SE); LAKE, Staffan, S-181 35 Lidingö (SE)
(74) Representative: Höglund, Lars
(86) International application number: PCT/SE2002/001268
(87) International publication number: WO 2002/103361

(56) References cited:
- WASKIEWICZ A.J. ET AL: 'Mitogen-activated protein kinases activate the serine/threonine kinases Mnk1 and Mnk2' THE EMBO JOURNAL vol. 16, no. 8, 1997, pages 1909 - 1920, XP002970663
- SCHEPER G.C. ET AL: 'The mitogen-activated protein kinase signal-integrating kinase Mnk2 is a eukaryotic initiation factor 4E kinase with high levels of basal activity in mammalian cells' MOL. CELL. BIOL. vol. 21, no. 3, February 2001, pages 743 - 754, XP002970664
- PYRONNET S.: 'Phosphorylation of the cap-binding protein eIF4E by the MAPK-activated protein kinase Mnk1' BIOCHEMICAL PHARMACOLOGY vol. 60, 2000, pages 1237 - 1243, XP002970665
- MAKKINJE A. ET AL: 'Phosphorylation of eukaryotic protein synthesis initiation factor 4E by insulin-stimulated protamine kinase' THE JOURNAL OF BIOLOGICAL CHEMISTRY vol. 270, no. 24, June 1995, pages 14824 - 14828, XP002970666
- FUKUNAGA R. ET AL: 'MNK1, a new MAP kinase-activated protein kinase, isolated by a novel expression screening method for identifying protein kinase substrates' THE EMBO JOURNAL vol. 16, no. 8, 1997, pages 1921 - 1933, XP002926236
- DATABASE MEDLINE US NATIONAL LIBRARY OF MEDICINE (NLM), BETHESDA, MD, US; 3 December 1998 (1998-12-03), COBB J.E. ET AL: "N-(2-Benzoylphenyl)-L-tyrosine PPAR gamma agonists. 3. Structure-actoivity relationship and optimization of the N-aryl substituent"

### SEQUENCE LISTING

<110> Biovitrum AB
<120> New methods
<130> 00572
<160> 9
<170> PatentIn version 3.0
<210> 1
   <211> 1444
   <212> DNA
   <213> human
<220>
   <221> CDS
   <222> (37)..(1434)
<300>
   <308> GenBank/AF237775
   <309> 2000-10-26
<400> 1
   cggtcccctc ccccgctggc ggggcccgga cagaag atg gtg cag aag aaa cca        54
                                           Met Val Gln Lys Lys Pro
                                           1               5
   gcc gaa ctt cag ggt ttc cac cgt tcg ttc aag ggg cag aac ccc ttc       102
   Ala Glu Leu Gln Gly Phe His Arg Ser Phe Lys Gly Gln Asn Pro Phe
               10                  15                  20
   gag ctg gcc ttc tcc cta gac cag ccc gac cac gga gac tct gac ttt       150
   Glu Leu Ala Phe Ser Leu Asp Gln Pro Asp His Gly Asp Ser Asp Phe
           25                  30                  35
   ggc ctg cag tgc tca gcc cgc cct gac atg ccc gcc agc cag ccc att       198
   Gly Leu Gln Cys Ser Ala Arg Pro Asp Met Pro Ala Ser Gln Pro Ile
       40                  45                  50
   gac atc ccg gac gcc aag aag agg ggc aag aag aag aag cgc ggc cgg       246
   Asp Ile Pro Asp Ala Lys Lys Arg Gly Lys Lys Lys Lys Arg Gly Arg
   55                  60                  65                  70
   gcc acc gac agc ttc tcg ggc agg ttt gaa gac gtc tac cag ctg cag       294
   Ala Thr Asp Ser Phe Ser Gly Arg Phe Glu Asp Val Tyr Gln Leu Gln
                   75                  80                  85
   gaa gat gtg ctg ggg gag ggc gct cat gcc cga gtg cag acc tgc atc       342
   Glu Asp Val Leu Gly Glu Gly Ala His Ala Arg Val Gln Thr Cys Ile
               90                  95                  100
   aac ctg atc acc agc cag gag tac gcc gtc aag atc att gag aag cag       390
   Asn Leu Ile Thr Ser Gln Glu Tyr Ala Val Lys Ile Ile Glu Lys Gln
           105                 110                 115
   cca ggc cac att cgg agc agg gtt ttc agg gag gtg gag atg ctg tac      438
   Pro Gly His Ile Arg Ser Arg Val Phe Arg Glu Val Glu Met Leu Tyr
       120                 125                 130
   cag tgc cag gga cac agg aac gtc cta gag ctg att gag ttc ttc gag      486
   Gln Cys Gln Gly His Arg Asn Val Leu Glu Leu Ile Glu Phe Phe Glu
   135                 140                 145                 150
   gag gag gac cgc ttc tac ctg gtg ttt gag aag atg cgg gga ggc tcc      534
   Glu Glu Asp Arg Phe Tyr Leu Val Phe Glu Lys Met Arg Gly Gly Ser
                   155                 160                 165
   atc ctg agc cac atc cac aag cgc cgg cac ttc aac gag ctg gag gcc      582
   Ile Leu Ser His Ile His Lys Arg Arg His Phe Asn Glu Leu Glu Ala
               170                 175                 180
   agc gtg gtg gtg cag gac gtg gcc agc gcc ttg gac ttt ctg cat aac      630
   Ser Val Val Val Gln Asp Val Ala Ser Ala Leu Asp Phe Leu His Asn
           185                 190                 195
   aaa ggc atc gcc cac agg gac cta aag ccg gaa aac atc ctc tgt gag      678
   Lys Gly Ile Ala His Arg Asp Leu Lys Pro Glu Asn Ile Leu Cys Glu
       200                 205                 210
   cac ccc aac cag gtc tcc ccc gtg aag atc tgt gac ttc gac ctg ggc      726
   His Pro Asn Gln Val Ser Pro Val Lys Ile Cys Asp Phe Asp Leu Gly
   215                 220                 225                 230
   agc ggc atc aaa ctc aac ggg gac tgc tcc cct atc tcc acc ccg gag      774
   Ser Gly Ile Lys Leu Asn Gly Asp Cys Ser Pro Ile Ser Thr Pro Glu
                   235                 240                 245
   ctg ctc act ccg tgc ggc tcg gcg gag tac atg gcc ccg gag gta gtg      822
   Leu Leu Thr Pro Cys Gly Ser Ala Glu Tyr Met Ala Pro Glu Val Val
               250                 255                 260
   gag gcc ttc agc gag gag gct agc atc tac gac aag cgc tgc gac ctg      870
   Glu Ala Phe Ser Glu Glu Ala Ser Ile Tyr Asp Lys Arg Cys Asp Leu
           265                 270                 275
   tgg agc ctg ggc gtc atc ttg tat atc cta ctc agc ggc tac ccg ccc      918
   Trp Ser Leu Gly Val Ile Leu Tyr Ile Leu Leu Ser Gly Tyr Pro Pro
       280                 285                 290
   ttc gtg ggc cgc tgt ggc agc gac tgc ggc tgg gac cgc ggc gag gcc      966
   Phe Val Gly Arg Cys Gly Ser Asp Cys Gly Trp Asp Arg Gly Glu Ala
   295                 300                 305                 310
   tgc cct gcc tgc cag aac atg ctg ttt gag agc atc cag gag ggc aag     1014
   Cys Pro Ala Cys Gln Asn Met Leu Phe Glu Ser Ile Gln Glu Gly Lys
                   315                 320                 325
   tac gag ttc ccc gac aag gac tgg gcc cac atc tcc tgc gct gcc aaa     1062
   Tyr Glu Phe Pro Asp Lys Asp Trp Ala His Ile Ser Cys Ala Ala Lys
               330                 335                 340
   gac ctc atc tcc aag ctg ctg gtc cgt gac gcc aag cag agg ctg agt     1110
   Asp Leu Ile Ser Lys Leu Leu Val Arg Asp Ala Lys Gln Arg Leu Ser
           345                 350                 355
   gcc gcc caa gtc ctg cag cac ccc tgg gtt cag ggg tgc gcc ccg gag      1158
   Ala Ala Gln Val Leu Gln His Pro Trp Val Gln Gly Cys Ala Pro Glu
       360                 365                 370
   aac acc ttg ccc act ccc atg gtc ctg cag agg aac agc tgt gcc aaa      1206
   Asn Thr Leu Pro Thr Pro Met Val Leu Gln Arg Asn Ser Cys Ala Lys
   375                 380                 385                 390
   gac ctc acg tcc ttc gcg gct gag gcc att gcc atg aac cgg cag ctg      1254
   Asp Leu Thr Ser Phe Ala Ala Glu Ala Ile Ala Met Asn Arg Gln Leu
                   395                 400                 405
   gcc cag cac gac gag gac ctg gct gag gag gag gcc gcg ggg cag ggc      1302
   Ala Gln His Asp Glu Asp Leu Ala Glu Glu Glu Ala Ala Gly Gln Gly
               410                 415                 420
   cag ccc gtc ctg gtc cga gct acc tca cgc tgc ctg cag ctg tct cca      1350
   Gln Pro Val Leu Val Arg Ala Thr Ser Arg Cys Leu Gln Leu Ser Pro
           425                 430                 435
   ccc tcc cag tcc aag ctg gcg cag cgg cgg caa agg gcc agt ctg tcc      1398
   Pro Ser Gln Ser Lys Leu Ala Gln Arg Arg Gln Arg Ala Ser Leu Ser
       440                 445                 450
   tcg gcc cca gtg gtc ctg gtg gga gac cac gcc tga ccctcccatc           1444
   Ser Ala Pro Val Val Leu Val Gly Asp His Ala
   455                 460                 465
<210> 2
   <211> 465
   <212> PRT
   <213> human
<400> 2
   Met Val Gln Lys Lys Pro Ala Glu Leu Gln Gly Phe His Arg Ser Phe
   1               5                   10                  15
   Lys Gly Gln Asn Pro Phe Glu Leu Ala Phe Ser Leu Asp Gln Pro Asp
               20                  25                  30
   His Gly Asp Ser Asp Phe Gly Leu Gln Cys Ser Ala Arg Pro Asp Met
           35                  40                  45
   Pro Ala Ser Gln Pro Ile Asp Ile Pro Asp Ala Lys Lys Arg Gly Lys
       50                  55                  60
   Lys Lys Lys Arg Gly Arg Ala Thr Asp Ser Phe Ser Gly Arg Phe Glu
   65                  70                  75                  80
   Asp Val Tyr Gln Leu Gln Glu Asp Val Leu Gly Glu Gly Ala His Ala
                   85                  90                  95
   Arg Val Gln Thr Cys Ile Asn Leu Ile Thr Ser Gln Glu Tyr Ala Val
               100                 105                 110
   Lys Ile Ile Glu Lys Gln Pro Gly His Ile Arg Ser Arg Val Phe Arg
           115                 120                 125
   Glu Val Glu Met Leu Tyr Gln Cys Gln Gly His Arg Asn Val Leu Glu
       130                 135                 140
   Leu Ile Glu Phe Phe Glu Glu Glu Asp Arg Phe Tyr Leu Val Phe Glu
   145                 150                 155                 160
   Lys Met Arg Gly Gly Ser Ile Leu Ser His Ile His Lys Arg Arg His
                   165                 170                 175
   Phe Asn Glu Leu Glu Ala Ser Val Val Val Gln Asp Val Ala Ser Ala
               180                 185                 190
   Leu Asp Phe Leu His Asn Lys Gly Ile Ala His Arg Asp Leu Lys Pro
           195                 200                 205
   Glu Asn Ile Leu Cys Glu His Pro Asn Gln Val Ser Pro Val Lys Ile
       210                 215                 220
   Cys Asp Phe Asp Leu Gly Ser Gly Ile Lys Leu Asn Gly Asp Cys Ser
   225                 230                 235                 240
   Pro Ile Ser Thr Pro Glu Leu Leu Thr Pro Cys Gly Ser Ala Glu Tyr
                   245                 250                 255
   Met Ala Pro Glu Val Val Glu Ala Phe Ser Glu Glu Ala Ser Ile Tyr
               260                 265                 270
   Asp Lys Arg Cys Asp Leu Trp Ser Leu Gly Val Ile Leu Tyr Ile Leu
           275                 280                 285
   Leu Ser Gly Tyr Pro Pro Phe Val Gly Arg Cys Gly Ser Asp Cys Gly
       290                 295                 300
   Trp Asp Arg Gly Glu Ala Cys Pro Ala Cys Gln Asn Met Leu Phe Glu
   305                 310                 315                 320
   Ser Ile Gln Glu Gly Lys Tyr Glu Phe Pro Asp Lys Asp Trp Ala His
                   325                 330                 335
   Ile Ser Cys Ala Ala Lys Asp Leu Ile Ser Lys Leu Leu Val Arg Asp
               340                 345                 350
   Ala Lys Gln Arg Leu Ser Ala Ala Gln Val Leu Gln His Pro Trp Val
           355                 360                 365
   Gln Gly Cys Ala Pro Glu Asn Thr Leu Pro Thr Pro Met Val Leu Gln
       370                 375                 380
   Arg Asn Ser Cys Ala Lys Asp Leu Thr Ser Phe Ala Ala Glu Ala Ile
   385                 390                 395                 400
   Ala Met Asn Arg Gln Leu Ala Gln His Asp Glu Asp Leu Ala Glu Glu
                   405                 410                 415
   Glu Ala Ala Gly Gln Gly Gln Pro Val Leu Val Arg Ala Thr Ser Arg
               420                 425                 430
   Cys Leu Gln Leu Ser Pro Pro Ser Gln Ser Lys Leu Ala Gln Arg Arg
           435                 440                 445
   Gln Arg Ala Ser Leu Ser Ser Ala Pro Val Val Leu Val Gly Asp His
   450 455 460
   Ala
   465
<210> 3
   <211> 1564
   <212> DNA
   <213> human
<220>
   <221> CDS
   <222> (37)..(1281)
<300>
   <308> GenBank/AF237776
   <309> 2000-10-26
<400> 3
   cggtcccctc ccccgctggc ggggcccgga cagaag atg gtg cag aag aaa cca       54
                                           Met Val Gln Lys Lys Pro
                                           1               5
   gcc gaa ctt cag ggt ttc cac cgt tcg ttc aag ggg cag aac ccc ttc      102
   Ala Glu Leu Gln Gly Phe His Arg Ser Phe Lys Gly Gln Asn Pro Phe
               10                  15                  20
   gag ctg gcc ttc tcc cta gac cag ccc gac cac gga gac tct gac ttt      150
   Glu Leu Ala Phe Ser Leu Asp Gln Pro Asp His Gly Asp Ser Asp Phe
           25                  30                  35
   ggc ctg cag tgc tca gcc cgc cct gac atg ccc gcc agc cag ccc att      198
   Gly Leu Gln Cys Ser Ala Arg Pro Asp Met Pro Ala Ser Gln Pro Ile
       40                  45                  50
   gac atc ccg gac gcc aag aag agg ggc aag aag aag aag cgc ggc cgg      246
   Asp Ile Pro Asp Ala Lys Lys Arg Gly Lys Lys Lys Lys Arg Gly Arg
   55                  60                  65                  70
   gcc acc gac agc ttc tcg ggc agg ttt gaa gac gtc tac cag ctg cag      294
   Ala Thr Asp Ser Phe Ser Gly Arg Phe Glu Asp Val Tyr Gln Leu Gln
                   75                  80                  85
   gaa gat gtg ctg ggg gag ggc gct cat gcc cga gtg cag acc tgc atc      342
   Glu Asp Val Leu Gly Glu Gly Ala His Ala Arg Val Gln Thr Cys Ile
               90                  95                  100
   aac ctg atc acc agc cag gag tac gcc gtc aag atc att gag aag cag      390
   Asn Leu Ile Thr Ser Gln Glu Tyr Ala Val Lys Ile Ile Glu Lys Gln
           105                 110                 115
   cca ggc cac att cgg agc agg gtt ttc agg gag gtg gag atg ctg tac      438
   Pro Gly His Ile Arg Ser Arg Val Phe Arg Glu Val Glu Met Leu Tyr
       120                 125                 130
   cag tgc cag gga cac agg aac gtc cta gag ctg att gag ttc ttc gag      486
   Gln Cys Gln Gly His Arg Asn Val Leu Glu Leu Ile Glu Phe Phe Glu
   135                 140                 145                 150
   gag gag gac cgc ttc tac ctg gtg ttt gag aag atg cgg gga ggc tcc        534
   Glu Glu Asp Arg Phe Tyr Leu Val Phe Glu Lys Met Arg Gly Gly Ser
                   155                 160                 165
   atc ctg agc cac atc cac aag cgc cgg cac ttc aac gag ctg gag gcc        582
   Ile Leu Ser His Ile His Lys Arg Arg His Phe Asn Glu Leu Glu Ala
               170                 175                 180
   agc gtg gtg gtg cag gac gtg gcc agc gcc ttg gac ttt ctg cat aac        630
   Ser Val Val Val Gln Asp Val Ala Ser Ala Leu Asp Phe Leu His Asn
           185                 190                 195
   aaa ggc atc gcc cac agg gac cta aag ccg gaa aac atc ctc tgt gag        678
   Lys Gly Ile Ala His Arg Asp Leu Lys Pro Glu Asn Ile Leu Cys Glu
       200                 205                 210
   cac ccc aac cag gtc tcc ccc gtg aag atc tgt gac ttc gac ctg ggc        726
   His Pro Asn Gln Val Ser Pro Val Lys Ile Cys Asp Phe Asp Leu Gly
   215                 220                 225                 230
   agc ggc atc aaa ctc aac ggg gac tgc tcc cct atc tcc acc ccg gag        774
   Ser Gly Ile Lys Leu Asn Gly Asp Cys Ser Pro Ile Ser Thr Pro Glu
                   235                 240                 245
   ctg ctc act ccg tgc ggc tcg gcg gag tac atg gcc ccg gag gta gtg        822
   Leu Leu Thr Pro Cys Gly Ser Ala Glu Tyr Met Ala Pro Glu Val Val
               250                 255                 260
   gag gcc ttc agc gag gag gct agc atc tac gac aag cgc tgc gac ctg        870
   Glu Ala Phe Ser Glu Glu Ala Ser Ile Tyr Asp Lys Arg Cys Asp Leu
           265                 270                 275
   tgg agc ctg ggc gtc atc ttg tat atc cta ctc agc ggc tac ccg ccc        918
   Trp Ser Leu Gly Val Ile Leu Tyr Ile Leu Leu Ser Gly Tyr Pro Pro
       280                 285                 290
   ttc gtg ggc cgc tgt ggc agc gac tgc ggc tgg gac cgc ggc gag gcc        966
   Phe Val Gly Arg Cys Gly Ser Asp Cys Gly Trp Asp Arg Gly Glu Ala
   295                 300                 305                 310
   tgc cct gcc tgc cag aac atg ctg ttt gag agc atc cag gag ggc aag       1014
   Cys Pro Ala Cys Gln Asn Met Leu Phe Glu Ser Ile Gln Glu Gly Lys
                   315                 320                 325
   tac gag ttc ccc gac aag gac tgg gcc cac atc tcc tgc gct gcc aaa       1062
   Tyr Glu Phe Pro Asp Lys Asp Trp Ala His Ile Ser Cys Ala Ala Lys
               330                 335                 340
   gac ctc atc tcc aag ctg ctg gtc cgt gac gcc aag cag agg ctg agt       1110
   Asp Leu Ile Ser Lys Leu Leu Val Arg Asp Ala Lys Gln Arg Leu Ser
           345                 350                 355
   gcc gcc caa gtc ctg cag cac ccc tgg gtt cag ggg tgc gcc ccg gag       1158
   Ala Ala Gln Val Leu Gln His Pro Trp Val Gln Gly Cys Ala Pro Glu
       360                 365                 370
   aac acc ttg ccc act ccc atg gtc ctg cag agg tgg gac agt cac ttc       1206
   Asn Thr Leu Pro Thr Pro Met Val Leu Gln Arg Trp Asp Ser His Phe
   375                 380                 385                 390
   ctc ctc cct ccc cac ccc tgt cgc atc cac gtg cga cct gga gga ctg      1254
   Leu Leu Pro Pro His Pro Cys Arg Ile His Val Arg Pro Gly Gly Leu
                   395                 400                 405
   gtc aga acc gtt act gtg aat gag tga agatcctgga ggaccctggg            1301
   Val Arg Thr Val Thr Val Asn Glu
               410
   ccccaggcca gctcccatcg ctgggggacg gtgaacggcc atgtgttaat gttacgatgt    1361
   ttttaaaaga caaaaaaaaa aaaaaaacct caaaagtttt tttaaagtgg gggaaaaaca    1421
   tccaagcact ttaattccaa tgtaccaggt gaactgacgg agctcagaag ttttccttta    1481
   caccaactgt caatgccgga attttgtatt ctgttttgta aagatttaat aaaagtcaaa    1541
   aaacttgcaa aaaaaaaaaa aaa                                            1564
<210> 4
   <211> 414
   <212> PRT
   <213> human
<400> 4
   Met Val Gln Lys Lys Pro Ala Glu Leu Gln Gly Phe His Arg Ser Phe
   1               5                   10                  15
   Lys Gly Gln Asn Pro Phe Glu Leu Ala Phe Ser Leu Asp Gln Pro Asp
               20                  25                  30
   His Gly Asp Ser Asp Phe Gly Leu Gln Cys Ser Ala Arg Pro Asp Met
           35                  40                  45
   Pro Ala Ser Gln Pro Ile Asp Ile Pro Asp Ala Lys Lys Arg Gly Lys
       50                  55                  60
   Lys Lys Lys Arg Gly Arg Ala Thr Asp Ser Phe Ser Gly Arg Phe Glu
   65                  70                  75                  80
   Asp Val Tyr Gln Leu Gln Glu Asp Val Leu Gly Glu Gly Ala His Ala
                   85                  90                  95
   Arg Val Gln Thr Cys Ile Asn Leu Ile Thr Ser Gln Glu Tyr Ala Val
               100                 105                 110
   Lys Ile Ile Glu Lys Gln Pro Gly His Ile Arg Ser Arg Val Phe Arg
           115                 120                 125
   Glu Val Glu Met Leu Tyr Gln Cys Gln Gly His Arg Asn Val Leu Glu
       130                 135                 140
   Leu Ile Glu Phe Phe Glu Glu Glu Asp Arg Phe Tyr Leu Val Phe Glu
   145                 150                 155                 160
   Lys Met Arg Gly Gly Ser Ile Leu Ser His Ile His Lys Arg Arg His
                   165                 170                 175
   Phe Asn Glu Leu Glu Ala Ser Val Val Val Gln Asp Val Ala Ser Ala
               180                 185                 190
   Leu Asp Phe Leu His Asn Lys Gly Ile Ala His Arg Asp Leu Lys Pro
           195                 200                 205
   Glu Asn Ile Leu Cys Glu His Pro Asn Gln Val Ser Pro Val Lys Ile
       210                 215                 220
   Cys Asp Phe Asp Leu Gly Ser Gly Ile Lys Leu Asn Gly Asp Cys Ser
   225                 230                 235                 240
   Pro Ile Ser Thr Pro Glu Leu Leu Thr Pro Cys Gly Ser Ala Glu Tyr
                   245                 250                 255
   Met Ala Pro Glu Val Val Glu Ala Phe Ser Glu Glu Ala Ser Ile Tyr
               260                 265                 270
   Asp Lys Arg Cys Asp Leu Trp Ser Leu Gly Val Ile Leu Tyr Ile Leu
           275                 280                 285
   Leu Ser Gly Tyr Pro Pro Phe Val Gly Arg Cys Gly Ser Asp Cys Gly
       290                 295                 300
   Trp Asp Arg Gly Glu Ala Cys Pro Ala Cys Gln Asn Met Leu Phe Glu
   305                 310                 315                 320
   Ser Ile Gln Glu Gly Lys Tyr Glu Phe Pro Asp Lys Asp Trp Ala His
                   325                 330                 335
   Ile Ser Cys Ala Ala Lys Asp Leu Ile Ser Lys Leu Leu Val Arg Asp
               340                 345                 350
   Ala Lys Gln Arg Leu Ser Ala Ala Gln Val Leu Gln His Pro Trp Val
           355                 360                 365
   Gln Gly Cys Ala Pro Glu Asn Thr Leu Pro Thr Pro Met Val Leu Gln
       370                 375                 380
   Arg Trp Asp Ser His Phe Leu Leu Pro Pro His Pro Cys Arg Ile His
   385                 390                 395                 400
   Val Arg Pro Gly Gly Leu Val Arg Thr Val Thr Val Asn Glu
                   405                 410
<210> 5
   <211> 28
   <212> DNA
   <213> Homo sapiens
<400> 5
   atggtgcaga agaaaccagc cgaacttc 28
<210> 6
   <211> 27
   <212> DNA
   <213> Homo sapiens
<400> 6
   gcccaggtcg aagtcacaga tcttcac 27
<210> 7
   <211> 49
   <212> DNA
   <213> Homo sapiens
<400> 7
   ggggacaagt ttgtacaaaa aagcaggctt cgtgcagaag aaaccagcc 49
<210> 8
   <211> 54
   <212> DNA
   <213> Homo sapiens
<400> 8
   ggggaccact ttgtacaaga aagctgggtc ctactcattc acagtaacgg ttct 54
<210> 9
   <211> 649
   <212> PRT
   <213> Homo sapiens
<220>
   <221> DOMAIN
   <222> (1)..(226)
<220>
   <221> DOMAIN
   <222> (237)..(649)
<400> 9
   Met Ala Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145 150 155 160
   Val Val Leu Tyr Met Asp Pro Met Cys Leu Asp Ala Phe Pro Lys Leu
   165 170 175
   Val Cys Phe Lys Lys Arg Ile Glu Ala Ile Pro Gln Ile Asp Lys Tyr
   180 185 190
   Leu Lys Ser Ser Lys Tyr Ile Ala Trp Pro Leu Gln Gly Trp Gln Ala
   195 200 205
   Thr Phe Gly Gly Gly Asp His Pro Pro Lys Ser Asp Leu Val Pro Arg
   210 215 220
   Ser Arg Ser Thr Ser Leu Tyr Lys Lys Ala Gly Phe Val Gln Lys Lys
   225 230 235 240
   Pro Ala Glu Leu Gln Gly Phe His Arg Ser Phe Lys Gly Gln Asn Pro
   245 250 255
   Phe Glu Leu Ala Phe Ser Leu Asp Gln Pro Asp His Gly Asp Ser Asp
   260 265 270
   Phe Gly Leu Gln Cys Ser Ala Arg Pro Asp Met Pro Ala Ser Gln Pro
   275 280 285
   Ile Asp Ile Pro Asp Ala Lys Lys Arg Gly Lys Lys Lys Lys Arg Gly
   290 295 300
   Arg Ala Thr Asp Ser Phe Ser Gly Arg Phe Glu Asp Val Tyr Gln Leu
   305 310 315 320
   Gln Glu Asp Val Leu Gly Glu Gly Ala His Ala Arg Val Gln Thr Cys
   325 330 335
   Ile Asn Leu Ile Thr Ser Gln Glu Tyr Ala Val Lys Ile Ile Glu Lys
   340 345 350
   Gln Pro Gly His Ile Arg Ser Arg Val Phe Arg Glu Val Glu Met Leu
   355 360 365
   Tyr Gln Cys Gln Gly His Arg Asn Val Leu Glu Leu Ile Glu Phe Phe
   370 375 380
   Glu Glu Glu Asp Arg Phe Tyr Leu Val Phe Glu Lys Met Arg Gly Gly
   385 390 395 400
   Ser Ile Leu Ser His Ile His Lys Arg Arg His Phe Asn Glu Leu Glu
   405 410 415
   Ala Ser Val Val Val Gln Asp Val Ala Ser Ala Leu Asp Phe Leu His
   420 425 430
   Asn Lys Gly Ile Ala His Arg Asp Leu Lys Pro Glu Asn Ile Leu Cys
   435 440 445
   Glu His Pro Asn Gln Val Ser Pro Val Lys Ile Cys Asp Phe Asp Leu
   450 455 460
   Gly Ser Gly Ile Lys Leu Asn Gly Asp Cys Ser Pro Ile Ser Thr Pro
   465                 470                 475                 480
   Glu Leu Leu Thr Pro Cys Gly Ser Ala Glu Tyr Met Ala Pro Glu Val
                   485                 490                 495
   Val Glu Ala Phe Ser Glu Glu Ala Ser Ile Tyr Asp Lys Arg Cys Asp
               500                 505                 510
   Leu Trp Ser Leu Gly Val Ile Leu Tyr Ile Leu Leu Ser Gly Tyr Pro
           515                 520                 525
   Pro Phe Val Gly Arg Cys Gly Ser Asp Cys Gly Trp Asp Arg Gly Glu
       530                 535                 540
   Ala Cys Pro Ala Cys Gln Asn Met Leu Phe Glu Ser Ile Gln Glu Gly
   545                 550                 555                 560
   Lys Tyr Glu Phe Pro Asp Lys Asp Trp Ala His Ile Ser Cys Ala Ala
                   565                 570                 575
   Lys Asp Leu Ile Ser Lys Leu Leu Val Arg Asp Ala Lys Gln Arg Leu
               580                 585                 590
   Ser Ala Ala Gln Val Leu Gln His Pro Trp Val Gln Gly Cys Ala Pro
           595                 600                 605
   Glu Asn Thr Leu Pro Thr Pro Met Val Leu Gln Arg Trp Asp Ser His
       610                 615                 620
   Phe Leu Leu Pro Pro His Pro Cys Arg Ile His Val Arg Pro Gly Gly
   625                 630                 635                 640
   Leu Val Arg Thr Val Thr Val Asn Glu
                   645

## Description

### TECHNICAL FIELD

The present invention relates to the use of the human "MAP kinase interacting kinases" Mnk2a and Mnk2b, in methods for identification of pharmaceutically useful agents, in particular agents useful for the treatment of type II diabetes.

### BACKGROUND ART

One of the major hormones that influences metabolism is insulin, which is synthesized in the beta cells of the islets of Langerhans of the pancreas. Insulin primarily regulates the direction of metabolism, shifting many processes toward the storage of substrates and away from their degradation (for reviews, see e.g. Shepherd, P.R. et al. (1998) Biochem. J. 333: 471-490; Alessi, D. R. & Downes, C. P. (1998) Biochim. Biophys. Acta 1436: 151-164). Insulin acts to increase the transport of glucose and amino acids as well as key minerals such as potassium, magnesium, and phosphate from the blood into cells. It also regulates a variety of enzymatic reactions within the cells, all of which have a common overall direction, namely the synthesis of large molecules from small units. A deficiency in the action of insulin (diabetes mellitus) causes severe impairment in (i) the storage of glucose in the form of glycogen and the oxidation of glucose for energy; (ii) the synthesis and storage of fat from fatty acids and their precursors and the completion of fatty-acid oxidation; and (iii) the synthesis of proteins from amino acids.

There are two varieties of diabetes. Type I is insulin-dependent diabetes mellitus (IDDM; formerly referred to as juvenile onset diabetes), for which insulin injection is required. In this type, insulin is not secreted by the pancreas and hence must be taken by injection. Type II diabetes, non-insulin-dependent diabetes mellitus (NIDDM), is characterized clinically by hyperglycemia and insulin resistance and is commonly associated with obesity. Type II diabetes is a heterogeneous group of disorders in which hyperglycemia results from both an impaired insulin secretory response to glucose and decreased insulin effectiveness in stimulating glucose uptake by skeletal muscle and in restraining hepatic glucose production (insulin resistance). Before diabetes develops, patients generally lose the early insulin secretory response to glucose and may secrete relatively large amounts of proinsulin. In established diabetes, although fasting plasma insulin levels may be normal or even increased in type II diabetes patients, glucose-stimulated insulin secretion is clearly decreased. The decreased insulin levels reduce insulin-mediated glucose uptake and fail to restrain hepatic glucose production.

Glucose homeostasis depends upon a balance between glucose production by the liver and glucose utilization by insulin-dependent tissues, such as fat and muscle, and insulin-independent tissues, such as brain and kidney. In type II diabetes, the entry of glucose into fat and muscle is reduced and glucose production in the liver is increased, due to insulin resistance in the tissues.

The receptor tyrosine kinases (RTKs) are a major type of cell-surface receptors. The ligands for RTKs are peptide/protein hormones including nerve growth factor (NGF), platelet-derived growth factor (PDGF), epidermal growth factor (EGF), and insulin. Binding of a ligand to an RTK stimulates the receptor's intrinsic protein-tyrosine kinase activity, which subsequently stimulates a signal-transduction cascade leading to changes in cellular physiology and patterns of gene expression. RTK signaling pathways have a wide spectrum of functions including regulation of cell proliferation and differentiation, promotion of cell survival, and modulation of cellular metabolism;

Ras is a GTP-binding switch protein that acts like a key signaling molecule in pathways triggered by activation of RTKs. All Ras-linked RTKs in mammalian cells appear to utilize a highly conserved signal-transduction pathway in which activated Ras induces a kinase cascade that culminates in the activation of MAP kinase (mitogen-activated protein kinase). This serine/threonine kinase, which can translocate into the nucleus, phosphorylates many different proteins including transcription factors that regulate expression of important cell-cycle and differentiation-specific proteins.

The murine Mnk1 and Mnk2 gene products ("MAP kinase interacting kinase" or "MAP kinase signal-integrating kinase" 1 and 2) are single-domain serine/threonine kinases that share 72% sequence identity (Waskiewicz A.J. et al. (1997) EMBO J. 16: 1909-1920; GenBank Accession Nos. Y11091 and Y11092). Human Mnk1 has also been described (Fukunaga, R. et al. (1999) EMBO J. 16: 1921-1933; GenBank Accession No. AB000409). All these three proteins were identified by their ability to bind tightly to MAP kinases. Both Mnk1 and 2 bind the extracellular signal-regulated kinases ERK1 and ERK2, and Mnk1 also binds the stress-activated kinase, p38. The eukaryotic initiation factor 4E (eIF4E) has been identified as one of the physiological substrates of Mnk1 and Mnk2 (Scheper, G.C. et al. (2001) Mol. Cell. Biol. 21: 743-754).

The human Mnk2 gene has been identified and characterized through a yeast two-hybrid screen in which the Mnk2 protein interacted with the ligand-binding domain of the estrogen receptor ϑ (ERβ) (Slentz-Kesler, K. et al. (2000) Genomics 69: 63-71). It was shown that the human Mnk2 gene has two C-terminal splice variants, designated Mnk2a (the nucleotide and amino acid sequences of Mnk2a are designated SEQ ID NOS:1 and 2, respectively; GenBank Accession No. AF237775) and Mnk2b (the nucleotide and amino acid sequences of Mnk2b are designated SEQ ID NOS: 3 and 4, respectively; GenBank Accession No. AF237776). The two isoforms are identical over the first 385 amino acids of the coding sequence and differ only in the final exon which encodes an additional 80 residues for Mnk2a and 29 residues for Mnk2b. It was further shown that the Mnk2 interaction was selective for estrogen receptor (ER)ϑ as opposed to ERI and that the interaction was specific to Mnk2b as opposed to Mnk2a or Mnk1.

### BRIEF DESCRIPTION OF THE DRAWINGS

Fig. 1 is a graph depicting the effect of Mnk2b overexpression in adipocytes 3T3-L1 transfected with GLU-REx3, CRE, IRE, or SREBP-RE. Control cells were transfected with an empty plasmid vector and the control expression level was set to 1.00.
Fig. 2 is a graph depicting the effect of Mnk2b on glucose uptake, when overexpressed in (2A) differentiated adipocytes 3T3-L1 and (2B) human neuronal cell line SHSY. Control cells (ctrl) were transfected with an empty plasmid vector. Grey staples indicate non-stimulated cells, white staples indicate insulin-stimulated cells.
Fig. 3 is a graph depicting the effect of Mnk2b overexpression and RNAi knock-down of Mnk2b expression on glucose uptake in human cells.

### DISCLOSURE OF THE INVENTION

It has surprisingly been found that Mnk2 is involved in the insulin-signaling pathway.

In one aspect, the invention features a method for identifying an agent that modulates (increases or decreases) the ability of an Mnk2 polypeptide to modulate glucose uptake in a cell, the method comprising: contacting an Mnk2 polypeptide with a candidate agent; and determining the effect of the candidate agent on the ability of the Mnk2 polypeptide to modulate glucose uptake in a cell. In one example, the candidate agent decreases the ability of the Mnk2 polypeptide to decrease glucose uptake in the cell.

In another aspect, the invention features a method for identifying an agent that modulates the ability of an Mnk2 polypeptide to modulate the activity of a glucose response element in a cell, the method comprising: contacting an Mnk2 polypeptide with a candidate agent; and determining the effect of the candidate agent on the ability of the Mnk2 polypeptide to modulate the activity of a glucose response element in a cell. In one example, the candidate agent decreases the ability of the Mnk2 polypeptide to decrease the activity of a glucose response element (e.g., a response element described herein) in the cell.

In another aspect, the invention features a method for identifying a modulator of glucose uptake, the method comprising: providing a cell expressing a recombinant Mnk2 polypeptide; exposing the cell to a candidate agent; and measuring glucose uptake in the cell in the presence of the candidate agent, wherein altered glucose uptake in the cell in the presence of the candidate agent compared to the absence of the candidate agent indicates that the candidate agent is a modulator of glucose uptake. In one example, the candidate agent causes increases glucose uptake.

A candidate agent can contain, for example, a peptide, peptidomimetic, amino acid, amino acid analog, polynucleotide, polynucleotide analog, nucleotide, nucleotide analog, or other small molecule. In one example, the candidate agent inhibits a Mnk2 biological activity such as a serine/threonine kinase activity, the ability to reduce glucose uptake in a cell, the ability to decrease activity of a glucose response element (e.g., an element described herein), and/or the ability to bind a Mnk2 ligand described herein. In one embodiment, the candidate agent binds to a Mnk2 polypeptide or a nucleic acid (RNA or DNA) encoding a Mnk2 polypeptide.

The screening methods described herein can optionally include a step of introducing into a cell a nucleic acid encoding a Mnk2 polypeptide. The effect of a candidate agent on a biological activity described herein can be evaluated in the presence and/or absence of a Mnk2 polypeptide or a nucleic acid encoding a Mnk2 polypeptide. The methods described herein can be carried out *in vitro* or *in vivo* using a cell-based system, a cell-free system, or a combination of cell-based and cell-free systems.

In another aspect, the invention features a method for modulating glucose uptake in vitro in a cell, the method comprising contacting a cell with an amount of a compound effective to modulate expression or activity of a Mnk2 polypeptide and thereby modulate glucose uptake in the cell.

The Mnk2 polypeptide used in the methods described herein.can be a mammalian Mnk2 polypeptide, e.g., a human Mnk2 polypeptide. For example, the Mnk2 polypeptide can be a human Mnk2a or Mnk2b polypeptide.

The Mnk2 polypeptide can have a sequence shown as SEQ ID NO:2 or SEQ ID NO:4. A Mnk2 polypeptide can also differ from the corresponding sequence shown as SEQ ID NO:2 or SEQ ID NO:4. The differences are, preferably, differences or changes at a non-essential residue or a conservative substitution. In one embodiment, the Mnk2 polypeptide includes an amino acid sequence at least about 60% identical to a sequence shown as SEQ ID NO:2 or SEQ ID NO:4 or a fragment thereof. Preferably, the amino acid sequence is at least 65%, 70%, 75%, 80%, 85%, 90%, 95%, 98%, 99% or more identical to SEQ ID NO:2 or SEQ ID NO:4 and has a Mnk2 biological activity described herein. For example, the amino acid sequence can be identical to SEQ ID NO:2 or SEQ ID NO:4.

Preferred Mnk2 polypeptides are at least 10%, preferably at least 20%, 30%, 40%, 50%, 60%, 70%, or more, of the length of the sequence shown as SEQ ID NO:2 or SEQ ID NO:4 and have a Mnk2 biological activity described herein. For example, a Mnk2 polypeptide can have a serine/threonine kinase activity, reduce glucose uptake in a cell, decrease activity of a glucose response element (e.g., an element described herein), and/or bind a Mnk2 ligand described herein.

A Mnk2 polypeptide also includes a polypeptide comprising a functional domain of the polypeptide of SEQ ID NO:2 or SEQ ID NO:4 described herein, e.g., a kinase domain. In one embodiment, the Mnk2 polypeptide has kinase activity.

A Mnk2 polypeptide also includes a polypeptide comprising at least 20, 30, 40, 50, 75, 100, 150, 200, 250, 300, 350, 400, 450, or more contiguous amino acid residues of SEQ ID NO:2 or SEQ ID NO:4. Preferably, the polypeptide has a Mnk2 biological activity described herein.

The Mnk2 polypeptide in some aspects of the invention can be a substantially pure polypeptide. The term "substantially pure" as used herein in reference to a given polypeptide means that the polypeptide is substantially free from other biological macromolecules. For example, the substantially pure polypeptide is at least 75%, 80, 85, 95, or 99% pure by dry weight. Purity can be measured by any appropriate standard method known in the art, for example, by column chromatography, polyacrylamide gel electrophoresis, or HPLC analysis.

Throughout this description the terms "standard protocols" and "standard procedures", when used in the context of molecular biology techniques, are to be understood as protocols and procedures found in an ordinary laboratory manual such as: Current Protocols in Molecular Biology, editors F. Ausubel et al., John Wiley and Sons, Inc. 1994, or Sambrook, J., Fritsch, E.F. and Maniatis, T., Molecular Cloning: A laboratory manual, 2nd Ed., Cold Spring Harbor Laboratory Press, Cold Spring Harbor, NY 1989.

Unless otherwise defined, all technical and scientific terms used herein have the same meaning as commonly understood by one of ordinary skill in the art to which this invention belongs. Suitable methods and materials are described below, although methods and materials similar or equivalent to those described herein can also be used in the practice or testing of the present invention. All publications, patent applications, patents, and other references mentioned herein are incorporated by reference in their entirety. In case of conflict, the present specification, including definitions, will control. In addition, the materials, methods, and examples are illustrative only and not intended to be limiting.

Below, the invention is described in the appended examples, which are intended to illustrate the invention, without limiting the scope of protection.

### EXAMPLES

### EXAMPLE 1: Identification of LK6 and Mnk

P element mediated mutagenesis is a widely used technology in *Drosophila* genetics (Cooley, L. et al. (1988) Science 239: 1121-1128; Robertson, H.M. et al. (1988) Genetics 118: 461-470). The P element is a well-characterized transposable element, which can introduce heritable loss of function mutations into a wide array of genes. Coupled with genomic annotation of the P element insertion site, P element libraries provide a valuable reverse genetics tool. Genetic screens using libraries of P insertion mutants in known genes enable a rapid scanning of the genome to identify potential modifier genes.

Impaired insulin receptor signaling has phenotypic manifestation of smaller cell size (Huang, H., et al. (1999) *PTEN affects cell size, cell proliferation and apoptosis during Drosophila eye development.* Development 126: 5365-5372.) A genetic screen was performed to identify modifiers of insulin receptor signaling, using a library of P insertion mutagenized *Drosophila* lines. In this screen, the small eye phenotypic manifestation was used as read out. The *D. melanogaster* gene LK6 (GenBank Accession No. U76378) was identified as a weak but consistent enhancer of the D.N. Insulin receptor phenotype.

The *Drosophila melanogaster* L6K protein was used in a TBLASTN search (http://www.ncbi.nlm.nih.gov/Education/blasttutorial.html) in the public nucleotide databases (http://www.ncbi.nlm.nih.gov/blast/). The human hits were MNK1 (AB000409; e-value of e-113), MNK2a (AF237775; e-value of e-114) and MNK2b (AF237776; e-value of e-110). Consequently, it was concluded by bioinformatic analysis that the *D. melanogaster* gene LK6 has two human homologues, Mnk1 and Mnk2.

### EXAMPLE 2: Cloning of MNK2b

The 3'-end of Mnk2b cDNA was isolated from Incyte clone No. 1309709. This clone contains a sequence corresponding to the last 570 bp of Mnk2b. The cDNA insert of the Incyte clone was verified by sequencing, performed by the ABI PrismBigDye Terminator Cycle Sequencing Ready Reaction Kit, on Applied Biosystems Model ABI 377 XL/96 DNA sequencing system.

To isolate a cDNA clone encoding the 5'-end of Mnk2b (670 bp), the public Mnk2b sequence (GenBank Accession No. AF237776) was used to design PCR primers for PCR amplification.

As template for cloning, cDNA made from human liver was used. A first strand cDNA synthesis, using SUPERSCRIPT Choice System (Life Technologies; Cat. # 18090-019) was performed using 1 µg human liver mRNA (Clontech; Cat. # 6510-1) with random hexamers according to the manufacturer's instructions.

The 100 µl PCR was performed using Native Pfu DNA Polymerase kit (Stratagene; Cat. # 600135) and 5 µl each of the gene specific primers LAKQ166 (SEQ ID NO: 5) and LAKQ168 (SEQ ID NO: 6). The Perkin Elmer DNA Thermal Cycler 480 was used with the program: 1 cycle of 94°C, 2 min; 60°C, 1 min; 74°C, 2 min; 25 cycles of 94°C, 1 min; 58°C, 1 min; 74°C, 2 min; and finally 72°C, 7 min followed by cooling to 4°C. Additional five rounds of amplification were performed as above, except that the annealing temperature was lowered to 55°C.

An aliquot, 15 µl, of the PCR was loaded on a 1% NuSieve GTG low melting temperature agarose gel (FMC BioProducts; Cat. #50082) and the fragment of about 670 bp was excised from the gel. 3 µl of the isolated fragment was cloned into 1 µl plasmid pCR2.1-TOPO using the TOPO TA Cloning Kit (Invitrogen; Cat. # K4500-01). 3 µl of the ligation mix was transformed into One Shot chemically competent TOP10 *E. coli* cells (Invitrogen; Cat. #C4040-03).

Plasmid DNA from three clones (3 ml overnight culture), were obtained by using the QIAprep Spin Miniprep Kit (QIAGEN; Cat. #27104) and the subsequent sequencing (#A0452) was performed as above. The plasmid with confirmed correct sequence was designated pMB 1500.

The two parts of Mnk2b, the 3'-end from Incyte1309709, and the 5'-end from pMB 1500, were joined together by a three-fragment ligation into pCR2.1-TOPO, yielding pBV1556.

2.4 µg of pBM1500 was digested with *Eco*RI and *Sac*I, and 1.8 µg of the same plasmid was digested with *Eco*RI and *Bgl*II. Half of the digestions were loaded on a 1.2% E-Gel, Invitrogen, and a band of approximately 3800 bp (fragment a) was cut out from the *Eco*RI*-Sac*I digestion, and a band of approximately 680 bp (fragment b) was cut out from the *Eco*RI*-Bgl*II digestion. 2.7 µg of the plasmid Incyte1309709 was digested with *Sac*I and *Bgl*II. Half of the digestion was loaded on an E-Gel and a band of approximately 700 bp (fragment c) was cut out from the gel. The fragments were purified using QIAquick Gel Extraction Kit (Qiagen; Cat. # 28704) and subsequent elution in 50 µl H₂O.

Ligation was performed using Ready-To-Go T4 DNA ligase (Amersham Pharmacia Biotech; Cat. # 27-0361-01). 6 µl of fragment A was mixed with 7 µl of fragments B and C, respectively. The ligation mix was transformed into One Shot chemically competent Top 10 *E. coli* cells, and plasmid DNA was obtained as above. Control digestions using the restriction enzymes used for cloning, *Eco*RI*, Sac*I and *Bgl*II verified the insert.

Mnk2b for mammalian expression was made by using Gateway^{™} Cloning Technology from Life Technologies. Gateway compatible primers were designed (SEQ ID NOS: 7 and 8) and PCR was performed using pBV1556 as a template. The PCR was performed in 50 µl using Taq DNA Polymerase, Roche (Cat. # 1 435 094) and 1 µl each of the primers BEKA 248 and BEKA247. The Perkin Elmer Gene Amp PCR system 2400 was used with the following program: 95°C, 5 min; (95°C 30 s, 55°C 30 s, 72°C 2 min) x 25; and 72°C, 7 min; followed by cooling to 4°C. 10 µl of the reaction was loaded on a 1.2% E-Gel, and a fragment of approximately 1400 bp was cut out from the gel, and purified using QIAquick Gel Extraction Kit.

The PCR fragment was cloned into pDONR201 (Life Technologies; Cat. # 11798-014), according to the manufacturer's instructions. The resulting entry clone was designated pBV27, and the insert was confirmed by sequencing. A mammalian expression clone with 5'-GST fusion, designated pBV44 (SEQ ID NO: 9), was constructed (according to the manufacturer's instructions) using the destination vector pDEST27 (Life Technologies; Cat. # 11812-013). In SEQ ID NO: 9, amino acids 1 to 226 represents the GST domain, while amino acids 237 to 649 represents human Mnk2b.

### EXAMPLE 3: Expression profiling

To determine the relative expression levels of MNK2b in different tissues, a Multiple Tissue Expression Array (CLONTECH; Cat. #775) was used in a hybridization experiment with a 106 bp gene specific probe.

The MNK2b cDNA clone, pBV27, was digested with the restriction enzymes *Nco*I and *Ppu*MI*.* The fragments were separated on a 1.2% agarose gel (Invitrogen; Cat. #G5018-01). The106 bp fragment was exercised from the gel and purified using QIA quick Gel Extraction Kit (QIAGEN; Cat. #28704).

25 ng of the purified fragment was used in a ³²P labeling reaction, performed with reagents as recommended in the Strip-EZ DNA probe synthesis instruction manual (Ambion; Cat. #1470). [α⁻³²P]dATP used in the reaction was purchased from Amersham Pharmacia Biotech (Cat. #AA0004).

Hybridization and washing conditions were performed as recommended by CLONTECH manual PT3307-1. The MTE Array was exposed in a STORM860 Phosphor Screen for 70 h. ImageQuant was used to analyze the hybridization signal.

The results indicated the highest expression levels for MNK2b in skeletal muscle. This was unexpected, since published results on adult mouse tissue showed expression of Mnk2 mRNA in all tissues studied, except for brain (Waskiewicz A.J. et al. (1997) EMBO J. 16: 1909-1920).

### EXAMPLE 4: Overexpression of Mnk2b affects glucose responsiveness and lipid metabolism in mouse adipocytes

Inducible reporter vectors that contain the *Photinus pyralis* (firefly) luciferase reporter gene, driven by a basic promoter element (TATA box), as well as inducible *cis-*enhancer elements (direct repeats from the promoter regions of various genes), were prepared or purchased. The reporter vectors are designed for the *in vivo* readouts of signal transduction pathways, since the enhancers are convergent points of many signal transduction pathways. When a plasmid expressing the gene of interest is cotransfected into mammalian cells with a cis-reporter plasmid, increased luciferase expression indicates either direct or indirect transcriptional activation.

A vector designated pGluREx3-Luciferase ((gtgCACGTGtgaCAGCTGcaa)x3) was prepared using the pTAL promoter vector (Clontech; cat. #6252). The pGluREx3 vector is designed to monitor effects on glucose response (Portois L., et al. (1999) J. Biol. Chem. 274: 8181-8190).

A vector designated pSREBP-Luciferase (aTCACcCCAC) was prepared by cloning two sterol regulatory element binding protein (SREBP) response elements into the pGLE2-promoter Vector (Promega; cat. #E1631). The pSREBP vector is designed to monitor effects on steroid response element (Yokyama, C. et al. (1993) Cell 75: 187-197).

The vector pCRE-Luciferase, designed to monitor the activation of cAMP binding protein (CREB) and cAMP-mediated signal transduction pathways, was purchased from Stratagene (cat. #219075).

A vector designated pIRE-Luciferase ((tagCAAAACAaactTATTTTGaaca)x3) was prepared, using the pGL2-Promoter Vector (Promega; cat. #E1631). The pIRE vector is designed to monitor insulin receptor mediated signaling through the insulin-like growth factor binding protein (IGFBP-1).

Mouse adipocytes (differentiated 3T3-L1 cells) were transiently transfected with the response element construct of interest, in combination with Mnk2b or a backbone (control) plasmid construct, using LipofectAmine™2000 (Life Technologies). After 48 hrs, the cells were lysed using a lysis buffer (Tris-EDTA + 0,25% Triton-X100) for 10 min at room temperature, and the luciferase activity was measured using a luciferase activity assay (BioThema).

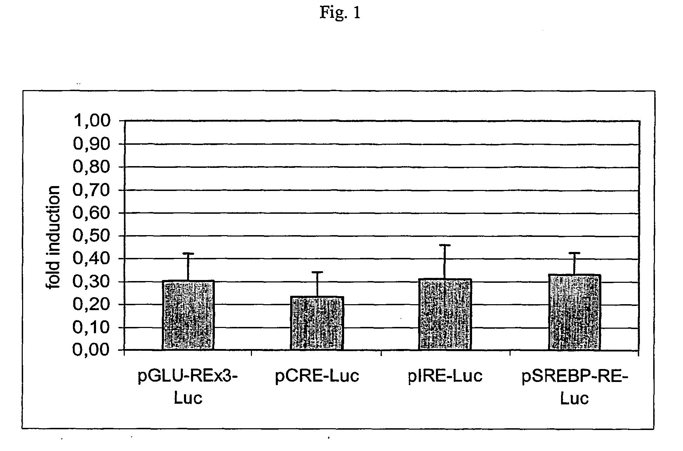

The results (Fig. 1) indicate that overexpression of Mnk2b in mouse adipocytes resulted in a 70% decrease of the activity of the GLUx3-Luciferase reporter, indicating a decrease in glucose responsiveness in the cells. To the inventors' knowledge, there are no previously disclosed results that indicate a link between Mnk2b and glucose uptake.

The results shown in Fig. 1 further indicate that overexpression of Mnk2b in mouse adipocytes leads to decreased activity of the SREBP response element. Our conclusion is that Mnk2b affects lipid metabolism, since the SREBP response element has been shown to control transcription of e.g. low density lipoprotein receptor gene (Yokoyama, C. et al. (1993) Cell 75: 187-197) and to regulate cholesterol metabolism (Brown, M.S. and Goldstein J.L. (1997) Cell 83: 331-340).

Overexpression of Mnk2b in mouse adipocytes also leads to decreased activity of the reporters pCRE and pIRE. These results confirm published data on Mnk2b as part of the MAP-kinase signaling pathway (Waskiewicz, A. et al. (1997) EMBO J. 16: 1909-1920; Fukunaga, R. and Hunter, T. (1997) EMBO J. 16: 1921-1933).

### EXAMPLE 5: Overexpression of Mnk2b modulates glucose uptake in adipocytes

Glucose uptake was determined according to the method of Hundal et al. (1994) Biochem. J. 297: 289-295. Briefly, after incubation with hormones for 45 minutes, if not otherwise stated, cell monolayers were rinsed with glucose free PBS. Glucose uptake was quantified by incubating the cells in the presence of 1 uCi/ml ³H-2-deoxy-glucose in PBS for 8 min. Non-specific uptake was determined by quantifying cell-associated radioactivity in the presence of 10 µM cytochalasin B. Uptake of 2-deoxy-glucose was terminated by rapidly aspirating the medium, followed by three successive washes of cell monolayers with ice cold PBS. The cells were lysed in 0.5 M NaOH, followed by liquid scintillation counting. Rates of transport were normalized for protein content in each well.

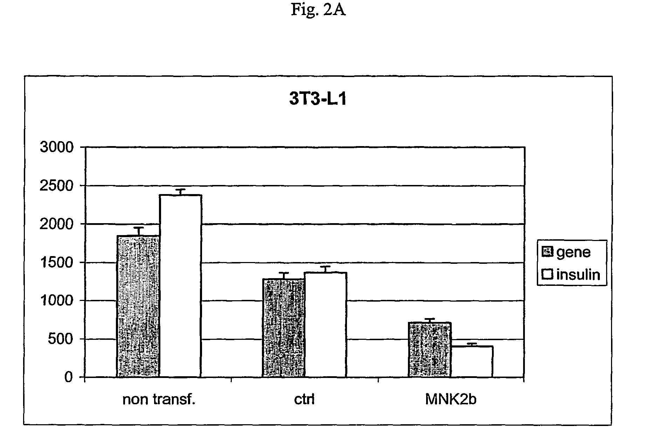

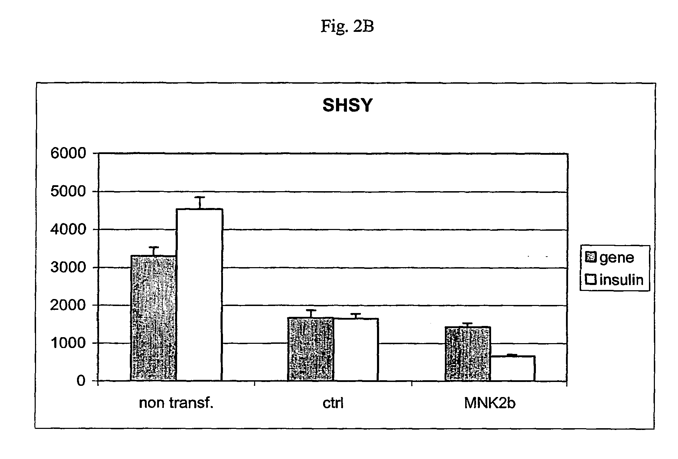

The results (Fig. 2) indicate that overexpression of Mnk2b in adipocytes (differentiated 3T3-L1 cells) and a human cell-line (SHSY) decreased the rate of glucose-uptake in an insulin-dependent manner. The results confirm the results from the reporter assay (Example 4) and further indicate that one effect of Mnk2b expression is reduction of glucose uptake.

### EXAMPLE 6: Structure models of Mnk proteins

Three-dimensional structure models of Mnk1, Mnk2a and Mnk2b were prepared from homology data. The structure of rat calmodulin-dependent protein kinase (Protein Data Bank entry 1A06) was used as template for all three models. (The Protein Data Bank is available at http://www.rcsb.org/pdb; see also Berman et al. (2000) Nucleic Acids Research 28: 235-242). The structure models were prepared using the ICM software from MolSoft Inc. (http://www.molsoft:com).

The models of Mnk1, Mnk2a and Mnk2b were highly similar but some structural differences were identified, which might be employed to achieve binding selectivity. A number of 87 non-identical residues were identified when Mnk2b was compared to Mnk1. Many of those are situated away from the active site, but two interesting differences between Mnk1 and Mnk2b are Y→H in the active site (cf. position 95 in SEQ ID NO: 4) and T→L in a loop that could be involved in substrate recognition (cf. position 248 in SEQ ID NO: 4).

A comparison between Mnk2a and Mnk2b indicated that the C-terminus, which is the only part differing between the two splice variants, folds against the active site in the models. This indicates the possible to identify agents having specificity between Mnk2a and Mnk2b.

### EXAMPLE 7: Knock-down of Mnk2 modulates glucose response in human neuroblastoma cells

RNAi (RNA interference) refers to the introduction of homologous double stranded RNA (dsRNA) to specifically target a gene's product, resulting in null or hypomorphic phenotypes. RNAi technique was used to study effects on glucose response in cultivated cells upon knock-down of Mnk2 protein expression. Human neuroblastoma (SH-SY5y) cells were transiently transfected with a glucose response element coupled to a luciferase reporter gene (GluREx3-Luciferase), Mnk2b or backbone plasmid and [RNAi-Mnk2] using LipofectAmine2000 (LifeTechnologies). For each well in a 96 well plate 0.2 µg GluREx3-Luciferase, 0.07 µg Mnk2b/backbone and 0.13 µg [RNAi-Mnk2] were mixed with 1.8 µl LA2000/ug DNA diluted in 50 µl Opti-MEM (Gibco). After 48 h. the cells were lysed using 15 µl/well lysis buffer (TRIS-EDTA with 0.25% Triton x100) and the luciferase activity was measured (Luciferase activity assay kit, BioThema).

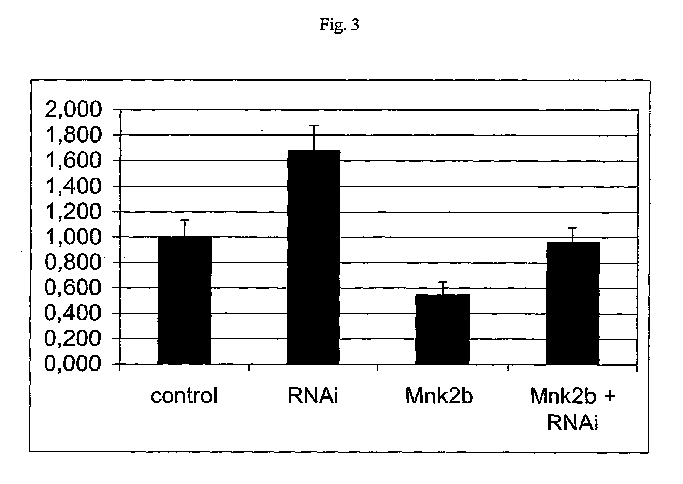

The results (Fig. 3) indicate that knock-down of Mnk2 protein expression in human neuroblastoma (SH-SY5y) cells by the use of RNAi leads to an increase in the activity of the glucose-response element. Over-expression of Mnk2b protein in the same cells, decreases the activity of the glucose-response element. This decrease is neutralized by knock-down of the over-expressed Mnk2b protein, that is combined transfection of expression plasmid and RNAi in the same cells.

### EXAMPLE 8: NMR screening for compounds binding Mnk2b

A diversity library consisting of relatively small and highly water-soluble compounds was used to screen native MNK-2B for binders by NMR (Nuclear Magnetic Resonance). The NMR technique used to identify binders was Saturation Transfer Difference (STD): the protein ¹H resonances are saturated by means of a weak radiofrequency field applied to a narrow spectral region. The saturation is transferred by spin diffusion to the rest of the protein and subsequently further to compounds that bind to the protein attenuating their signals in the NMR spectrum. The spectrum is then subtracted from a spectrum obtained at non-saturating conditions to obtain an STD spectrum showing only the signals from compounds interacting with the protein. In practice the pulse sequence is written in such a way that the subtraction is done automatically in every other scan, *i.e.* the individual spectra are never observed (Mayer & Meyer, *Angew. Chem. Int. Ed.,* **38**, 1784-1788, 1999).

The compounds in the library are divided into mixtures consisting of 4-8 compounds each. Each sample contained 1 µM native MNK-2B, 200 µM compound mixture, 50 mM sodium phosphate buffer, 1 mM DTT, pH 7.5 in ca 98% D₂O / 2% H₂O. One sample did not contain any compounds and functioned as a negative control. The volume was 600 µl and standard NMR tubes were used. Experiments were performed on a 600 MHz Varian Unity NMR spectrometer at 20°C. A reference ¹H 1D experiment and an STD experiment were recorded on each sample. The binders identified from the screen were rerun as a single compound for confirmation. For these follow-up experiments samples containing 2 µM native Mnk2B and 250 µM of the individual compound were used. Several compounds, for instance 4-hydroxy-benzoic acid methyl ester, were identified as Mnk2b ligands.

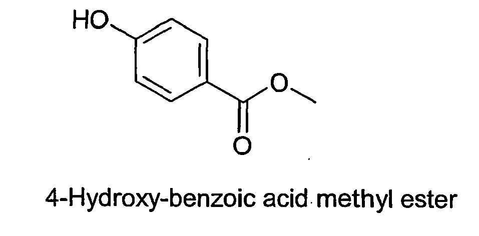

A kinase activity assay according to standard methods indicated the identified compounds inhibited Mnk2b kinase activity in a dose-dependent Consequently, it was shown that it is possible to identify small compounds Mnk2b ligands.

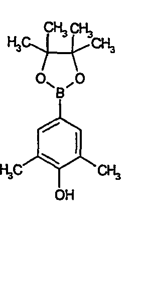

## Claims

1. A method for identifying an agent that modulates the ability of an Mnk2 polypeptide to modulate glucose uptake in a cell, the method comprising:
contacting an Mnk2 (MAP kinase interacting kinase 2) polypeptide with a candidate agent; and
determining the effect of the candidate agent on the ability of the Mnk2 polypeptide to modulate glucose uptake in a cell.

2. The method of claim 1, wherein the Mnk2 polypeptide is a mammalian Mnk2 polypeptide.

3. The method of claim 2, wherein the mammalian Mnk2 polypeptide is a human Mnk2 polypeptide.

4. The method of claim 2, wherein the mammalian Mnk2 polypeptide is Mnk2a.

5. The method of claim 4, wherein the Mnk2a polypeptide comprises the amino acid sequence of SEQ ID NO:2.

6. The method of claim 2, wherein the mammalian Mnk2 polypeptide is Mnk2b.

7. The method of claim 6, wherein the Mnk2b polypeptide comprises the amino acid sequence of SEQ ID NO:4.

8. A method for identifying an agent that modulates the ability of an Mnk2 polypeptide to modulate the activity of a glucose response element in a cell, the method comprising:
contacting an Mnk2 polypeptide with a candidate agent; and
determining the effect of the candidate agent on the ability of the Mnk2 polypeptide to modulate the activity of a glucose response element in a cell.

9. The method of claim 8, wherein the Mnk2 polypeptide is a mammalian Mnk2 polypeptide.

10. The method of claim 9, wherein the mammalian Mnk2 polypeptide is a human Mnk2 polypeptide.

11. The method of claim 9, wherein the mammalian Mnk2 polypeptide is Mnk2a.

12. The method of claim 11, wherein the Mnk2a polypeptide comprises the amino acid sequence of SEQ ID NO:2.

13. The method of claim 9, wherein the mammalian Mnk2 polypeptide is Mnk2b.

14. The method of claim 13, wherein the Mnk2b polypeptide comprises the amino acid sequence of SEQ ID NO:4

15. A method for identifying a modulator of glucose uptake, the method comprising:
providing a cell expressing a recombinant Mnk2 polypeptide;
exposing the cell to a candidate agent; and
measuring glucose uptake in the cell in the presence of the candidate agent, wherein altered glucose uptake in the cell in the presence of the candidate agent compared to the absence of the candidate agent indicates that the candidate agent is a modulator of glucose uptake.

16. An in vitro method for modulating glucose uptake in a cell, the method comprising contacting a cell with an amount of a compound effective to modulate expression or activity of a Mnk2 polypeptide and thereby modulate glucose uptake in the cell.

17. The method of claim 16, wherein the compound decreases expression or activity of the Mnk2 polypeptide and thereby increases glucose uptake in the cell.

18. The method of claim 17, wherein the compound decreases kinase activity of the Mnk2 polypeptide.

## Patentansprüche

1. Verfahren zum Identifizieren eines Mittels, welches die Fähigkeit eines Mnk2-Polypeptids moduliert, die Glucoseaufnahme in einer Zelle zu modulieren, wobei das Verfahren folgendes umfasst:
Kontaktieren eines Mnk2 (MAP-Kinase-interagierende Kinase 2)-Polypeptids mit einem Kandidatenmittel; und
Bestimmen des Effektes des Kandidatenmittels auf das Vermögen des Mnk2-Polypeptids, die Glucoseaufnahme in einer Zelle zu modulieren.

2. Verfahren gemäß Anspruch 1, wobei das Mnk2-Polypeptid ein Säuger-Mnk2-Polypeptid ist.

3. Verfahren gemäß Anspruch 2, wobei das Säuger-Mnk2-Polypeptid ein humanes Mnk2-Polypeptid ist.

4. Verfahren gemäß Anspruch 2, wobei das Säuger-Mnk2-Polypeptid Mnk2a ist.

5. Verfahren gemäß Anspruch 4, wobei das Mnk2a-Polypeptid die Aminosäuresequenz der SEQ ID Nr.: 2 umfasst.

6. Verfahren gemäß Anspruch 2, wobei das Säuger-Mnk2-Polypeptid Mnk2b ist

7. Verfahren gemäß Anspruch 6, wobei das Mnk2b-Polypeptid die Aminosäuresequenz der SEQ ID Nr.: 4 umfasst.

8. Verfahren zum Identifizieren eines Mittels, welches die Fähigkeit eines Mnk2-Polypeptids moduliert, die Aktivität eines Glucose-Response-Elementes in einer Zelle zu modulieren, wobei das Verfahren folgendes umfasst:
Kontaktieren eines Mnk2-Polypeptids mit einem Kandidatenmittel; und
Bestimmen des Effektes des Kandidatenmittels auf das Vermögen des Mnk2-Polypeptids, die Aktivität eines Glucose-Response-Elementes in einer Zelle zu modulieren.

9. Verfahren gemäß Anspruch 8, wobei das Mnk2-Polypeptid ein Säuger-Mnk2-Polypeptid ist.

10. Verfahren gemäß Anspruch 9, wobei das Säuger-Mnk2-Polypeptid ein humanes Mnk2-Polypeptid ist.

11. Verfahren gemäß Anspruch 9, wobei das Säuger-Mnk2-Polypeptid Mnk2a ist.

12. Verfahren gemäß Anspruch 11, wobei das Mnk2a-Polypeptid die Aminosäuresequenz der SEQ ID Nr.: 2 umfasst.

13. Verfahren gemäß Anspruch 9, wobei das Säuger-Mnk2-Polypeptid Mnk2b ist

14. Verfahren gemäß Anspruch 13, wobei das Mnk2b-Polypeptid die Aminosäuresequenz der SEQ ID Nr.: 4 umfasst.

15. Verfahren zum Identifizieren eines Modulators der Glucoseaufnahme, wobei das Verfahren folgendes umfasst:
Bereitstellen einer Zelle, die ein rekombinantes Mnk2-Polypeptid exprimiert;
Exponieren der Zelle an ein Kandidatenmittel; und
Messen der Glucoseaufnahme in der Zelle in Gegenwart des Kandidatenmittels, wobei die veränderte Glucoseaufnahme in der Zelle in Gegenwart des Kandidatenmittels im Vergleich zur Abwesenheit des Kandidatenmittels angibt, dass das Kandidatenmittel ein Modulator der Glucoseaufnahme ist.

16. In vitro-Verfahren zur Modulierung der Glucoseaufnahme in einer Zelle, wobei das Verfahren das Kontaktieren einer Zelle mit einer Menge an einer Verbindung, die zur Modulierung der Expression oder Aktivität eines Mnk2-Polypeptids wirksam ist und somit die Glucoseaufnahme in der Zelle moduliert, umfasst.

17. Verfahren gemäß Anspruch 16, wobei die Verbindung die Expression oder Aktivität des Mnk2-Polypeptids senkt und **dadurch** die Glucoseaufnahme in der Zelle erhöht.

18. Verfahren gemäß Anspruch 17, wobei die Verbindung die Kinaseaktivität des Mnk2-Polypeptids verringert.

## Revendications

1. Procédé d'identification d'un agent qui module la capacité d'un polypeptide Mnk2 à moduler l'absorption du glucose dans une cellule, le procédé comprenant :
la mise en contact d'un polypeptide Mnk2 (MAP kinase interacting kinase 2) avec un agent candidat ; et
la détermination de l'effet de l'agent candidat sur la capacité du polypeptide Mnk2 à moduler l'absorption du glucose dans une cellule.

2. Procédé selon la revendication 1, dans lequel le polypeptide Mnk2 est un polypeptide Mnk2 de mammifère.

3. Procédé selon la revendication 2, dans lequel le polypeptide Mnk2 de mammifère est un polypeptide Mnk2 humain.

4. Procédé selon la revendication 2, dans lequel le polypeptide Mnk2 de mammifère est Mnk2a.

5. Procédé selon la revendication 4, dans lequel le polypeptide Mnk2a comprend la séquence d'acides aminés de SEQ ID NO : 2.

6. Procédé selon la revendication 2, dans lequel le polypeptide Mnk2 de mammifère est Mnk2b.

7. Procédé selon la revendication 6, dans lequel le polypeptide Mnk2b comprend la séquence d'acides aminés de SEQ ID NO : 4.

8. Procédé d'identification d'un agent qui module la capacité d'un polypeptide Mnk2 à moduler l'activité d'un élément de réponse au glucose dans une cellule, le procédé comprenant :
la mise en contact d'un polypeptide Mnk2 avec un agent candidat ; et
la détermination de l'effet de l'agent candidat sur la capacité du polypeptide Mnk2 à moduler l'activité d'un élément de réponse au glucose dans une cellule.

9. Procédé selon la revendication 8, dans lequel le polypeptide Mnk2 est un polypeptide Mnk2 de mammifère.

10. Procédé selon la revendication 9, dans lequel le polypeptide Mnk2 de mammifère est un polypeptide Mnk2 humain.

11. Procédé selon la revendication 9, dans lequel le polypeptide Mnk2 de mammifère est Mnk2a.

12. Procédé selon la revendication 11, dans lequel le polypeptide Mnk2a comprend la séquence d'acides aminés de SEQ ID NO : 2.

13. Procédé selon la revendication 9, dans lequel le polypeptide Mnk2 de mammifère est Mnk2b.

14. Procédé selon la revendication 13, dans lequel le polypeptide Mnk2b comprend la séquence d'acides aminés de SEQ ID NO : 4.

15. Procédé d'identification d'un modulateur de l'absorption du glucose, le procédé comprenant :
la fourniture d'une cellule exprimant un polypeptide Mnk2 recombinant ;
l'exposition de la cellule à un agent candidat ; et
la mesure de l'absorption du glucose dans la cellule en présence de l'agent candidat, dans lequel l'absorption altérée du glucose dans la cellule en présence de l'agent candidat comparée à l'absence de l'agent candidat indique que l'agent candidat est un modulateur de l'absorption du glucose.

16. Procédé *in vitro* de modulation de l'absorption du glucose dans une cellule, le procédé comprenant la mise en contact d'une cellule avec une quantité d'un composé efficace pour moduler l'expression ou l'activité d'un polypeptide Mnk2 et ainsi moduler l'absorption du glucose dans la cellule.

17. Procédé selon la revendication 16, dans lequel le composé fait diminuer l'expression ou l'activité du polypeptide Mnk2 et fait ainsi augmenter l'absorption du glucose dans la cellule.

18. Procédé selon la revendication 17, dans lequel le composé fait diminuer l'activité kinase du polypeptide Mnk2.
